# EUROPEAN PATENT SPECIFICATION

(11) **EP 3 487 396 B1**
(45) Date of publication and mention of the grant of the patent: **01.01.2020**
(21) Application number: 18742800.8
(22) Date of filing: 20.07.2018
(51) Int. Cl.: A61B 5/00, A61B 5/053

(54) **WEARABLE DEVICE, SYSTEM AND METHOD**
WEARABLE-VORRICHTUNG, -SYSTEM UND -VERFAHREN
DISPOSITIF PORTABLE, SYSTÈME ET PROCÉDÉ

(30) Priority: 21.07.2017 EP 17182478
(43) Date of publication of application: 29.05.2019
(73) Proprietor: Koninklijke Philips N.V., 5656 AE Eindhoven (NL)
(72) Inventor: HUIJBREGTS, Laurentia, Johanna, 5656 AE Eindhoven (NL); HILGERS, Achim, 5656 AE Eindhoven (NL); AARTS, Ronaldus, Maria, 5656 AE Eindhoven (NL)
(74) Representative: Philips Intellectual Property & Standards
(86) International application number: PCT/EP2018/069752
(87) International publication number: WO 2019/016364

(56) References cited:
- WO-A1-2017/050784
- WO-A2-2016/003268
- US-A1- 2016 058 388
- US-A1- 2016 143 584

## Description

### FIELD OF THE INVENTION

The present invention relates to the field of wearable devices, such as fitness and activity trackers, and medical technology. In particular, the present invention relates to a wearable device adapted to be worn by a user, the wearable device comprising a fixation element for fixing the wearable device to the user and a lower side for contacting a skin of the user when worn. The present invention further relates to a method and corresponding computer program.

### BACKGROUND OF THE INVENTION

Vital signs of a person, for example the heart rate (HR), the respiration rate (RR) or the arterial blood oxygen saturation (SpO2), serve as indicators of the current health or fitness state of a person and as powerful predictors for serious medical events. For this reason, vital signs are extensively monitored in inpatient and outpatient care settings, at home or in further health, leisure and fitness settings.

During the recent years, wearable devices such as activity trackers, sports watches, and health watches have been developed. In particular, wrist-worn heart rate monitors have entered the market that measure e.g. the heart rate by using blood volume sensing techniques based on either photoplethysmography (PPG), bio-impedance or using a capacitive method to sense heart rate as described in US 8,260,405 B2.

Plethysmography generally refers to the measurement of volume changes of an organ or a body part and in particular to the detection of volume changes due to a cardiovascular pulse wave travelling through the body of a subject with every heartbeat. Photoplethysmography (PPG) is an optical measurement technique that evaluates a time-varying change of light reflectance or transmission of an area or volume of interest. PPG is based on the principle that blood absorbs light more than surrounding tissue, so that variations in blood volume with every heart beat affect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared), the blood oxygen saturation (SpO2) can be determined.

Wearable devices are usually worn on the arm or wrist. The sensors work best when they make good contact with the skin. Hence, the devices should preferably be strapped quite tightly. One reason why companies of PPG-based heart rate monitors advise their customers to strap the wearable device quite tightly around the arm is because at a higher pressure, the blood volume variations of the pulse of the arterial blood become stronger with respect to other blood volume variations (e.g. in veins and venules), and thereby the signal-to-noise ratio increases. Another reason to advise the customers to strap the device quite tightly is to avoid external light entering underneath the watch, thereby interfering with the signal.

WO 2016/097271 A2, an earlier application of the applicant, teaches that the contact pressure greatly influences the amplitudes of photoplethysmography and pulse oximetry signals. An apparatus is provided that comprises a physiological parameter sensor and an actuator for adjusting the pressure of contact between the physiological parameter sensor and the subject. The contact pressure is adjusted to improve the signal quality while maintaining contact between the sensor and the skin of the subject.

US 2016/0058388 A1 discloses a biosignal measuring method and apparatus. The biosignal measuring method includes verifying whether a measured biosignal is in a range, and controlling an operation of the biosignal measuring apparatus when the measured biosignal deviates from the range based on a result of the verifying.

WO 2017/050784 A1 discloses a strap-based wearable device for measuring a physiological parameter of a user. A sensor arrangement is used to convey information about the physiological parameter of the user. The tightness of the strap arrangement is controlled automatically in response to the quality of the sensor signals.

US 2016/0143584 A1 discloses a biological information measuring apparatus that includes a band which fixes a case unit to a living body. The band is provided with a recessed groove part on a side facing the living body. The groove part has a depth of 1020µm or more and 1140µm or less.

### SUMMARY OF THE INVENTION

It is an object of the present invention to provide a wearable device that enables improved wearing comfort and in particular assists in reducing skin irritation. It would further be advantageous to provide a wearable device that enables measurements with improved accuracy and/or reliability.

In a first aspect of the present invention a wearable device adapted to be worn by a user is presented. The wearable device comprises:
- a fixation element for fixing the wearable device to the user;
- a lower side for contacting a skin of the user when worn; and
- a processing unit adapted to determine a sweat level measure indicative of an amount of sweat accumulated between the lower side of the wearable device and the skin of the user; and to determine a moment in time for ventilating said lower side of the wearable device based on a said sweat level measure. Optionally the sweat level measure can further be indicative of an (accumulated) exposure to sweat over time, too.

In a further aspect of the present invention a system comprising a wearable device and a processing unit is presented. The wearable device is adapted to be worn by a user and comprises a fixation element for fixing the wearable device to the user; and a lower side for contacting a skin of the user when worn. The processing unit is adapted to determine a sweat level measure, indicative of an amount of sweat accumulated between the lower side of the wearable device and the skin of the user. The sweat level measure may further be indicative of a exposure to sweat over time. The processing unit is adapted to determine a moment in time for ventilating said lower side of the wearable device based on said sweat level measure.

In yet another aspect of the present invention, a method for operating a wearable device is presented. The wearable device comprises a fixation element for fixing the wearable device to a user, and a lower side for contacting a skin of the user when worn. The method comprises the steps of:
- fixing the wearable device to the user;
- determining, by a processing unit, a sweat level measure indicative of an amount of sweat accumulated between the lower side of the wearable device and the skin of the user; and
- determining, by the processing unit, a moment in time for ventilating said lower side of the wearable device based on a said sweat level measure.
Optionally, the sweat level measure can be indicative of an exposure to sweat over time, too.

In yet further aspects of the present invention, there are provided a corresponding computer program which comprises program code means for causing a computer to carry out the steps of determining a sweat level measure indicative of an amount of sweat accumulated between the lower side of a wearable device fixed to a user and the skin of the user; and determining a moment in time for ventilating said lower side of the wearable device based on said sweat level measure, when said computer program is carried out on a computer as well as a non-transitory computer-readable recording medium that stores therein a computer program product, which, when executed by a processor, causes said method steps disclosed herein to be performed.

Preferred embodiments of the invention are defined in the dependent claims. It shall be understood that the claimed system, method, computer program and medium can have similar and/or identical preferred embodiments as the claimed wearable device, in particular as defined in the dependent claims and as disclosed herein.

The present invention is based on the idea to ventilate a wearable device, more precisely, a lower side thereof in contact with a skin of the user, at appropriate moments in time. As explained above, in order to get reliable measurements, the sensors of wearable devices such as wrist watches or bracelets, need to make appropriate contact with the skin of the user. Hence, the device should preferably be strapped quite tightly. Additionally, in particular in a case of sensor using a light-based measurement with no proper mechanism to cope with ambient light, the housing or case around the sensor should also be light-tight. I.e., provide a light-tight seal with the skin of the subject, to avoid noise from external light. Due to the continuous contact of the device with skin, sweat cannot escape and will accumulate between the device and the skin, which may cause wearing discomfort and skin irritation.

A user may not recognize an accumulation of sweat, in particular if there is only a moderate accumulation of sweat, or if the user is distracted or sleeping. Nevertheless a sufficient amount of sweat may accumulate that can lead to skin irritation in particular when exposed to accumulated sweat over an extended period of time. During the day there can be a lot of motion and activity so that sweat may not accumulate naturally by device motion, i.e. involuntary repositioning to different skin portions. At night, when the user is sleeping or while the user engages in a sedentary activity, the wearable device may remain in a rather static state where sweat may accumulate which may lead to an unfavorable microclimate between a lower side of the wearable device and the skin of the user.

The solution proposed herein thus supports a user by providing a processing unit adapted to determining a sweat level measure indicative of an amount of sweat accumulated between the lower side of the wearable device and the skin of the user. In particular, the sweat level measure may be an indicative of an (accumulated or long-term) exposure to sweat over time. For example, the processing unit can be adapted to determine the sweat level measure based on an integration of a measurement signal, e.g. from at least one of a sweat sensor, a physiological parameter sensor, and an activity sensor over time, for example over a period of time of at least one of at least 5 minutes, at least 15 minutes, at least 1 hour, at least 2 hours, at least 4 hours, and at least a day. Instead of only evaluating an instantaneous sweat level or measurement condition, the long term wearing comfort may thus be improved and skin irritations may be avoided or at least reduced. The term sweat level measure may also refer to a sweat exposure level indicative of an exposure to sweat for a predetermined period of time. The sweat level measure may be determined based on an (estimated) amount of sweat as well as a duration of exposure to a sweat level. The sweat level measure may be determined based on an integral of an (estimated or measured) sweat level over time. For example, a lower sweat level may be tolerated over a longer period of time without causing skin irritation whereas a higher sweat level may require more frequent ventilation. The processing unit is further adapted to determine a moment in time for ventilating said lower side of the wearable device based on said sweat level measure. The processing unit can also be referred to as processor. The processing unit may be implemented by a single device, such as a microcontroller, application specific integrated circuit (ASIC) or field programmable gate array (FPGA), or by multiple devices exchanging data uni- or bidirectionally.

Based on said moment in time for ventilating said lower side of the wearable device, appropriate measures can be taken for ventilating said lower side of the wearable device to allow sweat to be removed, for instance to be evaporated. For example, air can be allowed to reach said lower side of the wearable device and/or skin of the subject and transport the moisture away. This concept can be seen similar to so-called air flow backpacks which enable ventilation between the backpack and the user's back. Regular backpacks make direct contact over large area of the (clothes covering the) back. Thereby, the user cannot get rid of his heat and sweat via the back. This might lead to feeling warm, wet clothes and salt spots on the clothes. Nonetheless, the situation with backpacks is different in that a continuous spacing may be provided since there is no need to make contact with the back of the user, e.g. in order to enable measurements of a physiological parameter of the subject. Hence, the solution proposed herein suggests a processing unit that, in a first step is adapted to determine a sweat level measure indicative of an amount of sweat accumulated between the lower side of the wearable device and the skin of the user; and, in a second step, to determine a moment in time for ventilating said lower side of the wearable device based on said sweat level measure.

Hence, a potentially critical condition can be identified that enables the user to react appropriately by either manually ventilating the device or by providing (active) means for ventilating said lower side of the wearable device.

As used herein, a wearable device can refer to an activity tracker or activity monitor, typically wrist-worn, but may also refer to a medical device at least a part of which is worn by the user for acquiring a physiological signal of the user. The wearable device can refer to a wearable monitoring apparatus for monitoring a physiological parameter of the user. The wearable device can comprise a physiological parameter sensor adapted to measure a physiological signal (indicative of a physiological parameter or vital sign) of the user.

A physiological signal of the user can refer to a physiological signal indicative of a vital sign or physiological parameter of the user. For instance, a physiological signal can be indicative of a photoplethysmography (PPG) measurement or a bio-impedance measurement, from which at least a physiological parameter or parameter indicative of a vital sign of the user can be derived such as, for example, a heart rate, respiration rate, blood oxygen saturation, galvanic skin response, electro-dermal activity or electrocardiogram (ECG).

A physiological parameter sensor for acquiring the physiological signal of the user can be adapted to acquire said physiological parameter of the user through contact with a part of the body of the user. The contact may be in direct contact between the physiological parameter sensor and the skin of the user or may be contact with the skin via some other medium. In particular, the physiological parameter sensor can be adapted to optically or electrically contact the skin of the user. The physiological parameter sensor is preferably arranged at the lower size of the wearable device.

Optionally, the wearable device can comprise a sweat sensor adapted to provide a sweat signal indicative of an amount of sweat of the user between said lower side of the wearable device and the skin of the user. The processing unit can be adapted to determine the sweat level measure based on said sweat signal. An advantage of this embodiment is that an accurate measurement of the actual sweat level can be derived to precisely determine an appropriate moment in time for ventilating said lower side of the wearable device. In other words, the wearable device can comprise a moisture sensing means for sensing a moisture level on the lower side of the wearable device.

Optionally, the wearable device can comprise at least one physiological parameter sensor for acquiring a physiological signal of the user. The processing unit can be adapted to determine the sweat level measure based on said physiological signal. An advantage of this embodiment is that a physiological parameter sensor which may already be provided within the wearable device for acquiring a physiological signal of the user can also be used to determine the sweat level measure. For example, a physiological parameter sensor may determine that the user is engaging in (intense) physical activity. A physical activity may in turn be correlated with increased sweat production. Hence, an estimation of the amount of sweat accumulated between the lower side of the wearable device and the skin of the user can be determined based thereon.

Optionally, in addition or in the alternative, the wearable device can comprise an activity sensor. The processing unit (20) can be adapted to determine the sweat level measure (32) based on an output of said activity sensor.

A sweat sensor and/or physiological parameter sensor can preferably be arranged on the lower side of the wearable device.

Optionally, the physiological parameter sensor, activity sensor (providing a signal indicative of an activity of the subject) or sweat sensor can comprise at least one of an electro-dermal activity sensor, a heart rate sensor, a motion sensor, and a temperature sensor, in particular a skin temperature sensor or core body temperature sensor. An electro dermal activity (EDA) sensor can also be referred to as a skin conductance or galvanic skin response (GSR) sensor. A sweat sensor as used herein may refer to a sensor for measuring a presence and/or an amount of sweat. The heart rate sensor may refer to any type of known heart rate sensor such as a photoplethysmography (PPG), bio-impedance or capacitance based sensor. Since an increased heart rate correlates with increased physical activity which in turn correlates with increased sweat production, a heart rate sensor can be used to determine the sweat level measure based on the heart rate of the user. An activity sensor can refer to a motion sensor, like an accelerometer or gyroscope. The temperatures sensor can advantageously determine a skin temperature since an increased skin temperature correlates with increased sweating or an environmental temperature sensor since there is also a correlation between environmental temperature and sweat production.

Optionally, the wearable device can comprise a timer (or clock). The processing unit can be adapted to determine the sweat level measure based on an output of said timer or clock. For example, a moment in time for ventilating said lower side of the wearable device can be determined based on a time interval since the placing the wearable device on the skin of the user, or a previous moment in time of ventilating the lower side of the wearable device. Hence, the sweat level measure can be determined based on or refer to a time elapsed since the last ventilation or a time interval since the placing the wearable device on the skin of the user. A wearing time may correlate with the amount of sweat accumulated underneath the wearable device. Optionally, a time of the day can be taken into consideration in order to ventilate the device more frequently when a higher sweat production is expected e.g. in the middle of the day or at noon or when the temperature is usually higher. Correspondingly, a lower frequency for ventilating the device can be determined when a sedentary activity of the user is expected such as in the evening or at night. Hence, the wearable device can comprise a timer and the processing unit can be adapted to determine an expected level of sweat as the sweat level measure indicative of an amount of sweat accumulated between the lower side of the wearable device and the skin of the user based on a time of the day and/or (expected) temporal activity pattern. Optionally, one or more sensor signals can be combined with the timer or clock.

Optionally, the wearable device can comprise one or more of a clock/timer, a motion sensor, a physiological parameter sensor, and a sweat sensor. The information from one or more of the clock/timer, the motion sensor, the physiological parameter sensor, and the sweat sensor can be used as an input for the processing unit to estimate the sweat level measure. The processing unit can thus be adapted to determine the sweat level measure based on an output of one or more of the clock/timer, the motion sensor, the physiological parameter sensor, and the sweat sensor.

Optionally, the processing unit can be adapted to determine a moment in time for ventilating said lower side of the wearable device based on said sweat level measure and further taking into account when a continuous measurement of a physiological parameter is (not) needed. For example, if the processing unit can be adapted to determine a moment of time under the condition that a physiological parameter is stable. On the other hand no ventilation is suggested if the physiological parameter varies, which may thus be indicative of an episode of high information content. Hence, a moment in time for ventilating said lower side of the wearable device may be postponed in order to acquire relevant physiological parameter information. On the other hand, if a stable period of a physiological parameter or indicative of low activity of the user or constant activity of the user is determined, such a period can be used for ventilating said lower side of the wearable device, i.e., only a limited amount of information would be lost. Hence, the processing unit can be adapted to determine the moment in time for ventilating said lower side of the wearable device based on said sweat level measure and optionally one or more of a parameter indicative of a stability and/or instability of a physiological parameter; a user/caregiver input, a calendar item (for example engaging in a sports activity or medical procedure where a continuous measurement is desired), a location information such as the user being in an operating room (where a continuous measurement is desired) or intensive care unit (ICU) or indicative of an activity of the user (such as an activity wherein a continuous measurement is desired).

Optionally, the wearable device can comprise an output unit, wherein the wearable device is further adapted to provide a notification about said moment in time for ventilating said lower side of the wearable device via said output unit. Hence, a user or caregiver can be instructed to take action for ventilating the device. The output unit can be part of the wearable device. Alternatively, in a system, the output can be part of an external entity such as a smartphone. The output unit can also be a communication interface of the wearable device.

Optionally, the wearable device is configured to adopt a ventilation state, wherein a spacing is provided between at least a portion of said lower side of the wearable device and the skin of the subject; and a contact state, wherein said portion of the lower side of the wearable device is configured to contact the skin of the subject. Hence, with said spacing in the ventilation state, an air gap can be provided for ventilating the lower side. The ventilation state can also be referred to as a sweat escape mode or state. The ventilation state preferably enables air flow to or ventilation of at least a portion of the lower side. The contact state, on the other hand, does not enable air flow to or ventilation of said portion. The wearable device can thus be configured to adopt two different states, selectively. The wearable device can be configured to change to said ventilation state based on said sweat level measure as determined by the processing unit at the moment in time for ventilating said lower side of the wearable device as determined by the processing unit. Once the lower side of the wearable device has been ventilated for sweat removal, the wearable device can again be configured to switch back to the contact state. For example, when using a timer, the wearable device can be configured to adopt the ventilation state when a first predetermined period of time has lapsed, for example, since the last displacement to the ventilation state or since first contact of the wearable device to the skin of the user. Correspondingly, the wearable device can be configured to resume to the contact state when a second predetermined period of time has lapsed since the last displacement to the ventilation state. For example, in the ventilation state, the wearable device may loosen the fixation element to provide a spacing, whereas in contact state the wearable device may tighten the fixation element for fixing the wearable device in contact with the user.

Optionally, the wearable device can be configured to oscillate between said ventilation state and said contact state when the processing unit determines the moment in time for ventilating said lower side of the wearable device. Thereby, the wearable device is adapted to actively remove moisture or sweat from between the lower side of the wearable device and the skin of the user. Hence, oscillating, i.e., alternating several times between said ventilation state and said contact state, can further improve sweat removal. The wearable device can be adapted to alternate at least twice between said ventilation state and said contact state when the processing unit determines the moment in time for ventilating said lower side of the wearable device. The wearable device may be adapted to pump sweat away by oscillating between said ventilation state and said contact state when the processing unit upon a single determination of a moment in time for ventilating said lower side of the wearable device.

Optionally, in a further refinement of this embodiment the wearable device can further comprise a physiological parameter sensor for acquiring a physiological signal of the user, wherein in said contact state the sensor is adapted to contact the skin of the user; and wherein in said ventilation state the sensor is adapted not to be in contact with the skin of the user. Hence, an area underneath the sensor, which can be considered to be the most relevant area for a measurement, can be ventilated.

Optionally, the physiological parameter sensor may switch to a different measurement mode or to a different sensor in said ventilation state than in said contact state. For example, a first measurement modality, such as photoplethysmography (PPG) measurement may be used when the physiological parameter sensor is in contact state, whereas a second, different measurement modality such a laser speckle imaging (LSI) may be used when the user is in ventilation state. The wearable device can be configured to use a measurement modality in the ventilation state that does not require contact to the skin of the user, i.e. a non-contact measurement of a physiological parameter. In an advantageous refinement, the physiological parameter sensor may be adapted to switch to a different measurement mode, e.g. contact or non-contact sensing mode using a same or different sensor such as PPG in contact mode and laser speckle imaging in non-contact mode.

Optionally, the wearable device can further comprise an actuator for (automatically) changing between said ventilation state and said contact state. The wearable device can comprise a controller for controlling said actuator based on the sweat level measure. For example, the sweat level can be determined using a sweat or moisture sensor and the wearable device can be configured to change to the ventilation state if said sweat level measure exceeds a first predetermined threshold. On the other hand, the wearable device can be configured to change to the contact state, if the sweat level falls below a second predetermined threshold. The first and second threshold can be the same or different. Preferably different thresholds are used wherein said second threshold is lower than said first threshold to provide a hysteresis. Thereby frequent changes between the two states can be avoided and an energy efficient implementation is provided to extend the battery life. Hence, the actuator can be adapted so as to enable ventilation of at least a portion of said lower side of the wearable device based on the sweat level measure at the moment in time for ventilating said lower side of the wearable device as determined by the processing unit.

Optionally, said actuator can comprise an electro-active material (EAM), in particular an electro-active polymer, EAP. In addition or in the alternative, a (micro) motor or other type of actuator can be used.

Optionally, the wearable device can comprise a spacing element adapted to provide said spacing between the wearable device and the skin of the subject, wherein the spacing element is displaceable between a first, ventilation state and a second, contact state, in particular wherein the spacing element is arranged between the lower side of the wearable and the skin of the user when worn. Optionally, in the first, ventilation state, the spacing element is at least partially distanced from the lower side of the wearable device, and in the second, contact state, the spacing element lies against the lower side of the wearable device. Hence, by providing said spacing and being distanced from the lower side of the wearable device in the ventilation state, the spacing element is adapted to enable a spacing to remove sweat from an area between the skin of the user and the lower side of the wearable device.

Optionally, the spacing element can be permeable to moisture. For example, the spacing element can comprise a membrane permeable to water and/or water vapor. The spacing element can comprise (micro-porous) PTFE (PolyTetraFluoroEthylene).

Optionally, the spacing element is a membrane and/or comprises at least one band or stripe.

Optionally, the wearable device further comprises a physiological parameter sensor for acquiring a physiological signal of the user, wherein the physiological parameter sensor is an optical physiological parameter sensor; and wherein the spacing element is transparent at a wavelength used by said optical physiological parameter sensor. More generally speaking, the sensor uses electromagnetic (EM) waves and the spacing element is transparent for a wave length used for the measurement. As used herein, transparent can refer to a transmission at a wavelength used by said optical physiological parameter sensor of more than 50%, preferably more than 70%, preferably more than 80%, preferably more than 90%. For example the physiological parameter sensor can be an optical physiological parameter sensor such as a PPG sensor or a laser speckle imaging sensor, or other sensor wherein light is used for physiological parameter measurement in particular to probe blood volume variations. Hence, a spacing can be provided between the skin of the user and said lower side of the wearable device while at the same time enabling the optical measurement.

Optionally, the actuator can comprise at least one displacement device at least partially made of an electro-active material, wherein the displacement device is mechanically connected to the spacing element for displacing the spacing element between the first, ventilation state and the second, contact state.

Optionally, at least one physiological parameter sensor can be integrated into the spacing element. Hence, ventilation between the skin of the user and a lower side of the wearable device can be provided while at the same time enabling continuous monitoring via the physiological parameter sensor. An advantage of this embodiment is that, for example if a strong signal-to-noise ratio is determined, at least a portion of the lower side of the wearable device can be ventilated (which may have otherwise provided shielding for the physiological parameter sensor) to enable ventilation of the lower side of the wearable device.

Optionally, in addition or in the alternative, the spacing element can at least partially be made of an electro-active material (electro-active polymer) and the actuator comprises a power supply for applying a voltage to the electro-active polymer. For example, the spacing element can thereby be configured to act as the actuator. The electro-active material of the spacing element may thus displace the spacing element such that the wearable device assumes the ventilation state when a first voltage is applied and to the contact state when a second voltage (or no voltage) is applied.

Referring to the embodiment of a system, it will be understood that the system may comprise a combination of a wearable device at another entity such as a smartphone or other external processing unit. The another entity can comprise the processing unit. Furthermore, the functionality of the processing unit can be partially implemented by the wearable device and partially implemented by another entity such as a smartphone. The wearable device and the other entity can be configured to communicate uni- or bidirectionally.

The invention is defined in the appended claims, the description with embodiments and examples serving for illustrative purpose.

### BRIEF DESCRIPTION OF THE DRAWINGS

These and other aspects of the invention will be apparent from and elucidated with reference to the embodiments described hereinafter. In the following drawings
Fig. 1 shows a schematic view of a first embodiment of a wearable device according to a first aspect of the present disclosure and a system according to the second aspect of the present disclosure;
Fig. 2 shows a side view of an embodiment of a wearable device in contact state;
Fig. 3 shows a first diagram of a physiological parameter and a sweat level measure over time;
Fig. 4 shows a second diagram of a physiological parameter and a sweat level measure over time;
Fig. 5 shows the wearable device of Fig. 2 in ventilation state;
Fig. 6 shows a further embodiment of a wearable device in ventilation state;
Fig. 7 shows an embodiment using a membrane as a spacing element;
Fig. 8 shows an embodiment of a wearable device comprising a micro-motor as an actuator;
Fig. 9 shows an embodiment of a wearable device comprising an electro-active actuator;
Fig. 10A and Fig. 10B illustrate the working principle of an electro-active material;
Fig. 11A and Fig. 11B shows different configurations of a spacing element;
Fig. 12 shows a side view of another embodiment of a wearable device in ventilation state;
Fig. 13A and 13B show a more detailed view of a contact state (Fig. 13A) and a ventilation state (Fig. 13B);
Fig. 14 shows a bottom view of the embodiment shown in Fig. 13A and Fig. 13B;
Fig. 15 shows an embodiment of a bi-stable element for providing a contact state and a ventilation state;
Fig. 16 shows a schematic flow chart of a method according to an aspect of the present disclosure.

### DETAILED DESCRIPTION OF THE INVENTION

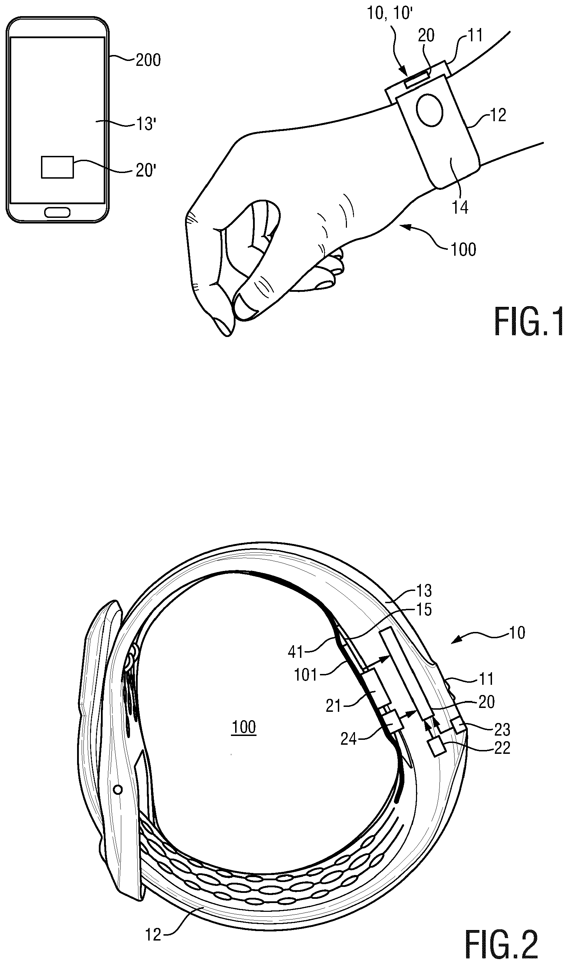

Fig. 1 schematically shows a first embodiment of a wearable device according to an aspect of the present disclosure. The wearable device is therein denoted in its entirety by reference numeral 10. In the given non-limiting example, the wearable device 10 is implemented in form of a wrist watch or activity tracker comprising a housing 11 and a fixation element 12. The housing of the wearable device 10 can comprise a battery, electronics, a communication interface, e.g. for communicating with a smartphone, and an optional output unit 13, for example in form of a display or speaker.

The wearable device 10 is adapted to be worn by a user 100. The wearable device 10 comprises a fixation element 12 for fixing the wearable device 10 to the user 100; and a lower side 15 for contacting a skin of the user when worn.

With photoplethysmography (PPG), bio-impedance or capacitive technology, contact sensors adapted to contact a skin of the user 100 and adapted to measure a physiological signal indicative of a vital sign (such as the heart rate of the user) can be integrated in wearable devices 10, 10' such as wrist watches or bracelets. In order to get reliable measurements, the sensors need to make appropriate contact with the skin and the device should preferably be strapped quite tightly. Additionally, and particular in case of light-based sensor with no proper mechanism to cope with ambient light, the case or housing around the sensor should also be light tight to avoid noise from external light. Since sweat cannot escape in prior art devices, and accumulates between the device and the skin, which may lead to wearing discomfort and skin irritation. If the device cannot move, this is in particular a problem in situations when wearable device is used during sports, which leads to excessive sweating. However, it has been found that even during non-strenuous activities and even during sleep sweat may accumulate and lead to wearing discomfort and skin irritation. Next to wearing discomfort and skin irritation, sweat accumulating between the skin and device might cause inaccurate readings.

Fig. 2 shows a side view of an embodiment of a wearable device 10 in contact state. According to a first aspect of the present disclosure, the wearable device 10 comprises a processing unit adapted to determine a sweat level measure indicative of an amount of sweat accumulated between the lower side 15 of the wearable device 10 and the skin 101 of the user 100; and to determine a moment in time for ventilating said lower side 15 of the wearable device 10 based on said sweat level measure.

According to a second aspect of the present disclosure, a system 1 is provided comprising a wearable device 10' adapted to be worn by a user 100 and a processing unit 20'. Hence, the processing unit can be part of the wearable device or part of another entity, thereby forming the system 1.For example, as shown in Fig. 1, the system 1 may comprise a combination of wearable device 10' and a smartphone 200 or other external processing unit 20'. The processing unit 20' of the system is adapted to determine a sweat level measure indicative of an amount of sweat accumulated between the lower side 15 of the wearable device 10' and a skin 101 of the user 100; and to determine a moment in time for ventilating said lower side 15 of the wearable device 10' based on said sweat level measure. Optionally, the wearable device 10' may comprise a communication interface for communicating with an external device such as a smartphone 200 comprising said (external) processing unit 20'. The (external) processing unit 20' can take similar and/or identical embodiments as the (internal) processing unit 20. To avoid repetitions, the following examples will thus only refer to the case of an (internal) processing unit 20.

An internal output unit 13 or external output unit 13' can be provide to provide a notification about said moment in time for ventilating said lower side 15 of the wearable device 10. For example, the wearable device can provide one or more of a visual output via a display or light source, an audible output via a speaker, and/or a tactile output via a vibration unit. Correspondingly, such output means can be provided by the smartphone 200 or other external entity.

The wearable device 10 can comprise at least one physiological parameter sensor 21 for acquiring a physiological signal 31 of the user. For example, the physiological parameter sensor can be a photoplethysmography (PPG) sensor or bio-impedance sensor for measuring a heart rate of the user. The processing unit 20 can advantageously be adapted to determine the sweat level measure based on said physiological signal. An increased heart rate indicates that the user is engaging in physical activity and may therefore have an increased sweat production such that a larger amount of sweat is produced per time unit. Hence, the physiological signal provides an indication of the amount of sweat accumulated between the lower side of the wearable device and the skin of the user. Based thereon, an appropriate moment in time for ventilating said lower side of the wearable device can be determined. In addition or in the alternative, the wearable device can comprise a timer 22. The processing unit can be adapted to determine the sweat level measure based on an output of said timer 22.

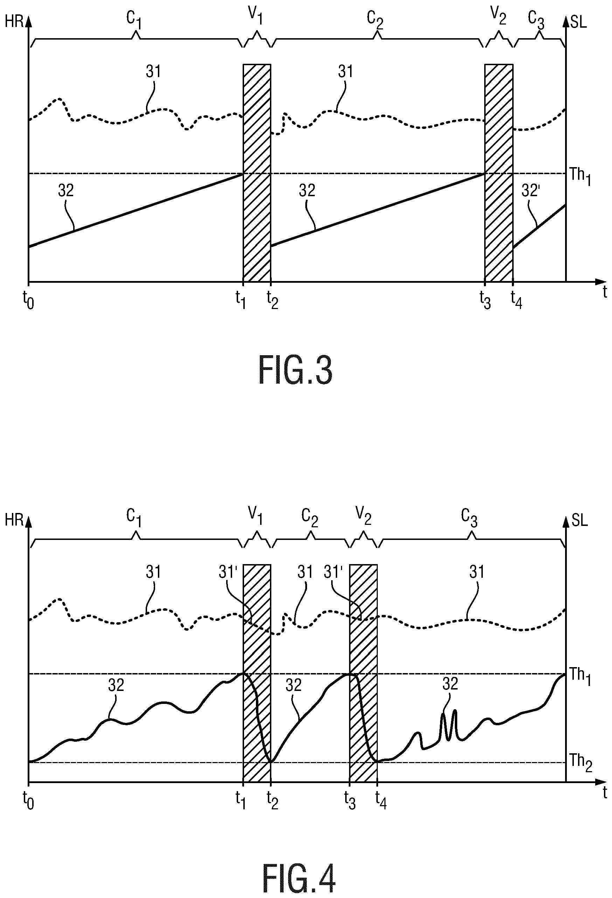

Fig. 3 shows a diagram of a physiological parameter 31, here the heart rate, over time. The vertical axis denotes the time t. The left vertical axis denotes the physiological parameter, here the heart rate HR. The right vertical axis denotes a sweat level measure SL.

Conventional wearable devices often provide a continuous physiological signal trace 31. The solution as proposed herein, however, is based on the idea to ventilate the wearable device, and in particular a lower side 15 thereof in contact with the skin 101 of the subject 100 when worn, at appropriate moments in time. As indicated in Fig. 3, the measurement is divided into a first section C₁ wherein the lower side 15 contacts the skin of the user such that the physiological parameter sensor 21 (cf. Fig. 2) can provide a physiological signal 31. This is also referred to as contact mode. The following section, indicated by V₁, denotes a period of time for ventilating said lower side of the wearable device. Hence, the physiological parameter sensor may be lifted and no longer be in contact with the skin of the user. Air may reach at least a portion of said lower side, thereby enabling sweat removal by ventilation. The measurement is interrupted and no physiological signal 31 is acquired during V₁.

After this period, the wearable device may again operate in contact mode indicated by period C₂ wherein the physiological signal 31 can again be acquired. This period may again be followed by a second ventilation period V₂, and so on.

The processing unit 10 is adapted to determine a sweat level measure 32 indicative of an amount of sweat accumulated between the lower side of the wearable device and the skin of the user. In the embodiment shown in Fig. 3, sweat level measure is determined based on the output of the timer 22 (cf. Fig. 2). In a basic configuration as illustrated in Fig. 3, it can be assumed that the sweat level measure 32 increases linearly over time. A moment in time t1 for ventilating said lower side of the wearable device can be determined based on said sweat level measure 32, for example as a moment in time when the sweat level measure 32 exceeds a threshold Th₁. In Fig. 3, the threshold Th₁ is indicated by the dashed horizontal line.

In an optional refinement, as indicated in the segment C₃, a slope of said sweat level measure 32 can be adapted on additional measurements. For example, the wearable device 10, as illustrated in Fig. 2, may further comprise a temperature sensor 23 and said sweat level measure indicative of the amount of sweat accumulated between the lower side of the wearable device and the skin of the user can be determined based on an output of said timer 22 and said temperature sensor 23. For example, a slope of the sweat level measure 32 can be adjusted based on the temperature, since an increased temperature is expected to lead to increased sweat production.

Referring again to Fig. 2, the wearable device 10 can, in addition or in the alternative, comprise a sweat sensor 24 adapted to provide a sweat signal indicative of an amount of sweat of the user between said lower side 15 of the wearable device 10 and the skin 101 of the user 100. The processing unit 20 can be adapted to determine the sweat level measure based on said sweat signal. For example, the sweat level measure can correspond to the sweat signal. Optionally, the physiological parameter sensor 21 can serve as the sweat sensor 24.

Fig. 4 shows a graph similar to Fig. 3 wherein the sweat level measure 32 indicative of an amount of sweat accumulated between the lower side of the wearable device and the skin of the user is now determined based on a sweat signal provided by the sweat sensor 24 (cf. Fig. 2). The processing unit 20 can be adapted to determine a moment in time for ventilating said lower side of the wearable device based on said sweat level 32, for example by determining when said sweat level 32 exceeds a first predetermined threshold Th₁. Correspondingly, the processing unit 20 can be adapted to determine a moment in time for ending said ventilating of the lower side of the wearable device. For example, it can be determined when said sweat level measure 32 falls below a second predetermined threshold Th₂ as indicated by the horizontal dashed line in Fig. 4. Advantageously different predetermined thresholds Th₁ and Th₂ are used to provide a hysteresis, thereby limiting the amount of switching between a ventilation state and a contact state.

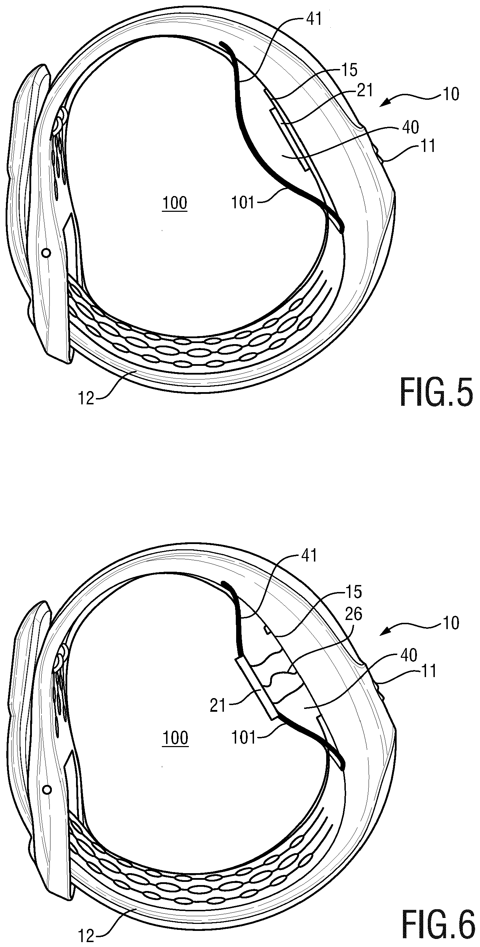

Referring now to Fig. 5, the wearable device 10 is advantageously configured to adapt a ventilation state, wherein a spacing 40 is provided between at least a portion of said lower side 15 of the wearable device and the skin 101 of the subject 100; and a contact state, as shown in Fig. 2, wherein said portion of the lower side 15 of the wearable device 10 is configured to contact the skin 101 of the subject 100. The spacing 40 enables an airflow to or ventilation of at least a portion of the lower side 15 as shown in Fig. 5. Hence, referring again to Fig. 3, the wearable device can be configured to adapt a contact state (as shown in Fig. 2) during the periods Cₓ, and assume a ventilation state as illustrated in Fig. 5, during the periods indicated by Vₓ.

In the embodiment shown in Fig. 5, the physiological parameter sensor 21 is provided at the lower side 15 of the wearable device 10. Hence, when the spacing 40 is provided, a measurement of the physiological signal 31 may be interrupted as also indicated during the periods Vₓ in Fig. 3. Hence, in some embodiments of the present disclosure, the physiological parameter monitoring can be interrupted when the device gets in a ventilation state, also referred as 'sweat-escape mode', i.e. the mode when the ventilation is taking place. Hence, a non-continuous measurement may be provided such that data may be lost. However, the advantages of continuous and near- or quasi-continuous monitoring have to be weighted. For example, long-term patterns such as a sleep pattern, daily heart rate pattern, etc. even if interrupted for a limited period of time of, for example, a few seconds in between, can still provide valuable information regarding an overall pattern. Furthermore, the information that is acquired during contact mode but without disturbances due to sweating may have a superior signal quality which may outweigh the drawbacks of having a non-continuous monitoring. Hence, information missed while the sweat is being evaporated may be negligible when compared to data that is received for the whole night along with less skin irritation. Hence, in said contact state the sensor can be adapted to contact the skin of the user (as shown in Fig. 2) and in said ventilation state (as shown in Fig. 5), the sensor can be adapted not to be in contact with the skin 101.

As a solution to overcome the drawback of having a non-continuous monitoring, the physiological parameter sensor can be adapted to operate in a first, contact mode, for example providing a photoplethysmography measurement when being in contact mode, and to switch to a different measurement during ventilation state, for example using laser speckle imaging. Thereby, a first physiological signal 31 can be acquired in contact mode and a second physiological signal 31' can be provided in ventilation mode (cf. signal traces 31 and 31' in Fig. 4). Advantageously, this can efficiently be implemented by using a coherent light source both for laser speckle imaging and photoplethysmography such that the different measurement mode may be affected by using a different signal processing of the output signal of a photodetector of the physiological parameter sensor. In the alternative, the physiological parameter sensor can comprise a first sub-sensor for contact based measurement and a second sub-sensor for measurement in non-contact mode.

In order to change between said contact state (as shown in Fig. 2) said ventilation state (as shown in Fig. 5), an actuator can be provided for changing between said ventilation state and said contact state, and a controller for controlling said actuator based on the sweat level measure, in particular based on the moment in time for ventilating said lower side of the wearable device as determined by the processing unit 20 (cf. Fig. 2).

In the embodiments shown in Fig. 2 and 5, a spacing element 41 is provided. The spacing element is adapted to provide said spacing 40 between the wearable device 10 and the skin 101 of the subject 100. The spacing element 41 is displaceable between a first, ventilation state (as shown in Fig. 5) and a second, contact state (as shown in Fig. 2). In the shown embodiment, the spacing element 41 is arranged between the lower side 15 and the skin 101 of the user 100 when worn. In the first, ventilation state, the spacing element is at least partially distanced from the lower side 15 of the wearable device 10, as shown in Fig. 5, and in the second, contact state, the spacing element 41 lies against the lower side 15 of the wearable device, as shown in Fig. 2.

In the shown embodiment, the spacing element 41 is implemented in form of a membrane that is permeable to moisture and thereby enables evaporation of sweat from the skin 101 of the subject via the spacing 40 as shown in Fig. 5. The spacing 40 can be created by applying a tension to the membrane 41. If the spacing 40 is created between the skin 101 and the lower side 15 of wearable device 10, ventilation can take place via the open area, thereby evaporating the sweat.

It has been found that such extra tension (pressure on the skin) during a ventilation state can be tolerable since the time spent in ventilation state may be limited. Advantageously, the fixation element 12 comprises a portion made of an elastomer adapted to elongate upon providing an increased tension, for example moving the device by 1-2 mm.

Fig. 6 shows an embodiment, wherein the physiological parameter sensor 21 moves with the spacing element 41. For example, the physiological parameter sensor 21 can be attached to the membrane. An advantage of this embodiment is that continuous monitoring of a physiological parameter can be provided. For example, the processing unit can further be adapted to determine the moment in time for ventilating said lower side of the wearable device based on said sweat level measure and also a signal quality of the physiological parameter sensor 21, for example a signal to noise ratio. If a high signal to noise ratio is determined, a higher level of background noise may be tolerated such that a spacing 40 can be created between the skin 101 of the subject 100 and the lower side 15 of the wearable device 10. The physiological parameter sensor 21 may be connected via electrical connections 26.

In Figs. 5 und 6, the creation of the spacing 40 is schematically illustrated in an exaggerated manner. In practice, a smaller spacing can be sufficient.

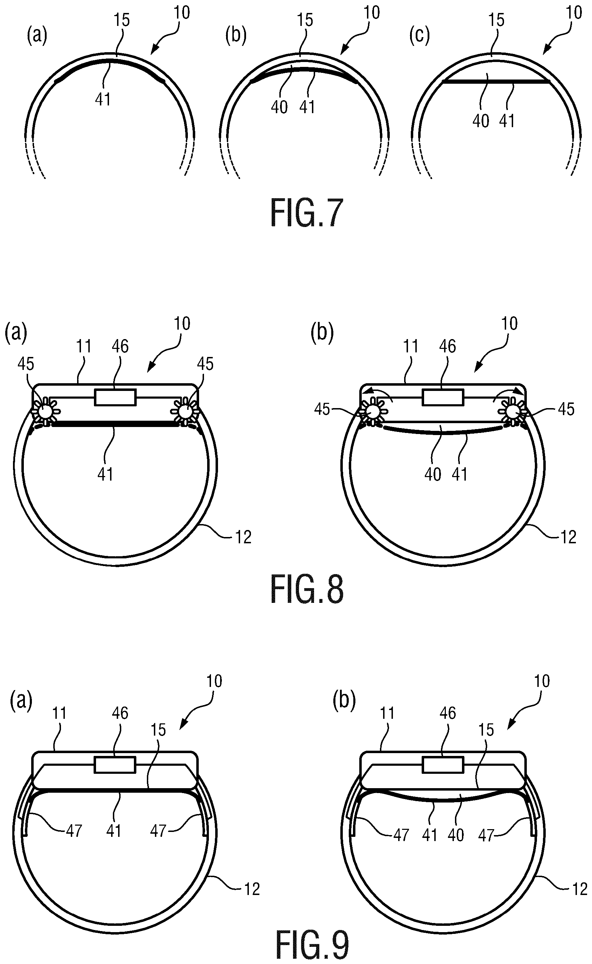

Fig. 7 illustrates three different situations of different tension of a membrane as the spacing element 41. In Fig. 7(a) the membrane 41 is relaxed and thus in contact with a lower side 15 of the wearable device 10. In Fig. 7(b) the membrane 41 is tensioned so as to create a small spacing 40, whereas a larger spacing 40 is provided in Fig. 7(c) by further increasing the tension on the membrane 41. In practice, it can be advantageous to implement a solution as illustrated in Fig. 7(c) wherein the membrane 41 may be flat or may even have some curvature as shown in Fig. 7(b) rather than implementing a situation where the membrane gets deflected as schematically illustrated in Fig. 5 and Fig. 6.

Fig. 8 illustrates a first embodiment of an actuator 45 for changing between said ventilation state, as shown in Fig. 8(b), and said contact state, as shown in Fig. 8(a). A controller 46 is provided for controlling said actuator 45 based on the sweat level measure. The processing unit 20 and the controller 46 can be implemented as different units or as a single unit, for example by a microcontroller. In the embodiment shown in Fig. 8, one or more micro-motors 45 are used to tension the spacing element in form of the membrane 41. For example, a tooth-wheel like configuration can be applied for the motor to engage with the membrane.

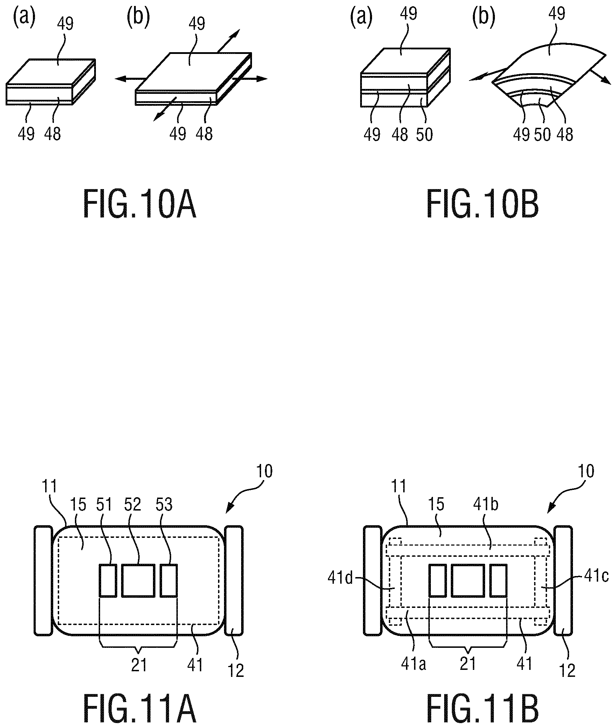

In the preceding embodiment described with reference to Fig. 7, a (relatively) soft membrane may be used. In the embodiment shown in Fig. 8, the membrane 41 has a predetermined stiffness such that the membrane 41 is adapted to be pushed together and may bend downwards, thereby lifting the wearable device 10 from the skin and ventilating the lower side of the wearable device. Because the lower side of the wearable device provides a rigid boundary, the membrane 41 is not able to bend upwards and therefore slightly compresses the skin of the subject but only temporarily during ventilation. The membrane 41 can be a foil with openings or a porous foil having a predetermined stiffness. In case of using an electroactive material, such as an electro-active polymer (EAP) as will be described further below with reference to Fig. 9 and Figs. 10A and 10B, the same principle applies. By applying an electrical voltage to the EAP the components starts to e.g. expand, i.e. its lateral dimensions are increasing. If opposite endpoints are fixed or clamped, for example at the wrist band 12 or the ends of the housing 11, the membrane 41 attached to the EAP will bend downwards, as illustrated in Fig. 9(b).

In an alternative embodiment as shown in Fig. 9, an electro-active material, EAM, in particular an electro-active polymer, EAP, can be provided. For the underlying working principle of an electro-active device comprising an electro-active polymer reference is made to Fig. 10A and Fig 10B.

Electro-active polymers (EAP) are an emerging class of materials within the field of responsive materials. EAPs can easily be manufactured into various shapes allowing easy integration. EAP actuators can directly transduce an input energy to mechanical work. EAP actuators can be subdivided in field-driven and ionic-driven actuators.

Fig. 9(a) shows an embodiment of a field-driven EAP actuator comprising an electro-active polymer 46 sandwiched between two (flexible) electrodes 47. By the electrodes 47 an electric field can be applied across the electro-active polymer 46. EAPs usually require high fields (volts per meter) but low currents due to their capacitive nature. Polymer layers 46 are preferably thin to keep the driving voltage as low as possible. Prominent examples of field driven EAPs are piezoelectric and electro-strictive polymers as well as dielectric elastomers. Other examples include electro-strictive graft polymers, electro-strictive paper, electrets, electro-viscoelastic elastomers and liquid crystal elastomers.

It has been found that the limited electromechanical performance of traditional piezoelectric polymers can be further improved by polyvinylidene (di-)fluoride (PVDF) relaxor polymers which provide an advantageous spontaneous electric polarization (field driven alignment). These materials can also be pre-strained for improved performance in the strained direction since pre-strain leads to better molecular alignment. Normally, metal electrodes can be used since strains can be in a moderate regime (1-5 %). Other types of electrodes 47 such as conducting polymers, carbon black based oils, gels or elastomers, etc. can also be used. The electrodes 47 can be continuous or segmented.

Fig. 10A shows a configuration of a field-driven EAP where the polymer 46 is sandwiched between two compliant electrodes 47. Fig. 10B illustrates a bending actuator, wherein a carrier layer 48 is combined with an EAP, thereby making a bi- or multi-layer configuration. The EAP film can be stretched (molecular orientation) by applying a voltage via the electrodes 47, which forces the bending in one (preferred) direction as illustrated in Fig. 10B(b).

Fig. 9 thus shows an alternative embodiment, wherein an electro-active actuator is used, in particular comprising an electro-active polymer (EAP). For example, instead of using micro-motors as shown in Fig. 8, one or more electro-active materials such as electro-active polymers 47 at one or more sides of the membrane 41 can be used to create the tension. In the embodiment shown in Fig. 9, one EAP is connected at each side of the membrane 41. An outer side of each EAP 47 can be fixed to the fixation element 12, here in form of a wrist band. In an activated state, as shown in Fig. 9(a) the EAPs can be shrunk and thus stretching the membrane. If the EAPs are deactivated, as shown in Fig. 9(b), they may expand and generate a pressure towards the membrane which can be deflecting accordingly thereby creating a spacing 40 at a lower side 15 of the wearable device 10. A controller 46 can be provided for controlling the EAP actuators 47 based on the sweat level measure 32, in particular (de-)activating the EAP actuators 47 at a moment in time for ventilating said lower side 15 of the wearable device 10 as determined by the processing unit 20 (cf. Fig. 2).

In another embodiment, the membrane 41 itself can be made of an electro-active material.

Advantageously, the EAP can be arranged in a so-called clamped arrangement wherein the EAP is fixed at at least two (opposite) sides, e.g. at two sides of the lower side 15 of a housing 11 of the wearable device 10. If the EAP is activated, the deformation can be as indicated in Fig. 10B(b). Optionally, a ring-clamped configuration is possible, wherein the EAP can be fully clamped along its circumference, at least at a plurality of points along its circumference.

Fig. 11A and Fig. 11B show bottom views of two embodiments of a wearable device 10 comprising a housing 11 and a fixation element 12. A physiological parameter sensor 21 is arranged at a lower side 15 of a housing 11 of the wearable device 10. The physiological parameter sensor 21 can be a PPG sensor comprising a first light source 51, a detector 52 and optionally a second light source 53. The second light source 53 can be adapted to emit light at a different wavelength than the first light source 51, such that a blood oxygen measurement can be performed.

The embodiment shown in Fig. 11A corresponds to the case shown in Fig. 5, wherein the physiological parameter sensor 21 is arranged at the lower side 15. In order to provide an optical measurement, the spacing element, here in form of a membrane 41 covering the lower side of the wearable device 10 is transparent at a wavelength used by said optical physiological parameter sensor 21. Alternatively, the spacing element may comprise one or more holes or openings to provide a light path for the optical measurement, i.e., for the light coming from the light source 51 and/or 53 towards the skin of the user and going back to the detector 52 of the physiological parameter sensor 21.

Optionally, an EAP can be used that is made from a transparent material such as e.g. disclosed in US 7 969 645 B2 or Samuel Shian, et al. ("High-speed, compact, adaptive lenses using in-line transparent dielectric elastomer actuator membranes") in combination with ITO-layers as metal electrodes or grid-like electrode configurations. Accordingly, the EAP may be (optically) transparent to a physiological parameter sensor, here a PPG sensor which may be embedded in the lower side of the wearable device 10. Holes can be provided to enable evaporation of sweat.

In an embodiment, the EAP can be covered by a standard plastic material. The plastic material can serve as a carrier layer wherein the physiological parameter sensor 21 can be fixed to the EAP forming the spacing element as already illustrated in Fig. 6. For example, the physiological parameter may be fixed, e.g. soldered, onto electrical footprints and/or tracks on the electric active polymer itself. For example, metal solder dots and tracks can be implemented on a plastic cover of the active material of the electro-active polymer. This approach can be considered similar to flex-foil printed circuits boards.

In an embodiment, the spacing element 41 can comprise (vertical) holes between the lower side 15 of the wearable device and the skin 101 of the subject 100 when worn. An advantage of this approach is improved air ventilation. Also a diaphragm-like EAP configuration is possible. Optionally, holes can be provided as a controllable valve structure adapted to a let a fluid (or gas) flowing through said opening from one side to the other. The valve may be open if the EAP is activated and may be closed if the EAP is deactivated. In yet another embodiment, the EAP may comprise small holes which may be switched from an open to a close state. In the open state, ventilation can take place.

In addition or as an alternative to providing an at least partially transparent spacing element 41, the spacing element 41 may have an opening for the physiological parameter sensor 21.

Referring to Fig. 11B, the spacing element 41 can optionally comprise different portions 41a-41d. Each portion may comprise an electro-active polymer. Optionally, the portions 41a and 41b or also further sections can alternately be activated or deactivated, for example in a cascaded manner, to support sweat removal, e.g. providing a cascaded motion for sweat removal. For example, the sections can alternate activated or deactivated, for instance, a first set of strips is activated in parallel during a first time and a second set of stripes is deactivated, and vice versa during a second period of time. For example all even EAP sections are activated while the uneven EAP sections are deactivated during a first time frame, and during a next time frame, the status may be reversed to opposite states.

Referring to a comparison of Figs. 5 and 6, when the physiological parameter sensor 21 is moving with the spacing element 41, as shown in Fig. 6, the spacing element 41 does not need to be transparent and the physiological measurement can continue to take place even in ventilation state or sweat-escape mode. However, the configuration shown in Fig. 5 has advantages regarding stability and manufacturing. However, as already indicated in the measurement trace of Fig. 3, the physiological parameter sensor may not be in contact with the skin of the user during ventilation state and therefore a signal-to-noise ratio of the physiological parameter measurement may become too high and thereby a measurement may have to be neglected during ventilation state as illustrated by time intervals Vₓ in Fig. 3.

Referring again to Fig. 3, the membrane as the spacing element 41 may be tensioned at regular time intervals. A timer 22 or clock may thus be used as a trigger. In particular, the membrane may be tensioned when a sweat sensor 24 (cf. Fig. 2) measures that there is a substantial amount of sweat, i.e. exceeding a predetermined threshold. A state of the art galvanic skin response sensor can be used as the sweat sensor 24. In other embodiments, one or more other sensors may be used to estimate the amount of sweat. For example, one or more of a skin temperature sensor, an environment temperature sensor, a motion sensor such as an accelerometer or gyroscope, or a heart rate sensor which could be the physiological parameter sensor 21 of the device like a PPG, bio-impedance or capacitance sensor. Sweat excursion is expected when high temperatures are measured, when the heart rate is high and/or when an activity-like running or cycling is recognized from a motion sensor signal. In such periods of expected perspiration, the wearable device could be changed between ventilation state and contact state every now and then, i.e. when a moment in time for ventilating said lower side of the wearable device is determined by the processing unit.

After the wearable device has switched to ventilation state, it can be switched back to contact state when no ventilation is needed anymore, i.e., because the sweat has evaporated. The timing of switching back to contact state can either be based on a timer (e.g. a predetermined time such as one minute after switching to ventilation state), on a sensor that gives a sweat measure such as a galvanic skin response sensor or one or more sensors from which sweat production is estimated (e.g. skin temperature, heart rate, or motion sensor, in combination with an estimate of how much sweat has evaporated, which can be based on the amount of time in the ventilations state).

Optionally, the ventilation state may be switched off manually, for example in cases where continuous monitoring is needed, for example when the user is doing a VO₂-max test or when knowledge about a maximum heart rate during a dedicated measurement time interval is desired. Thereby, it is ensured that no relevant measurement data is lost during an undesired occurrence of a ventilation state. For example, the ventilation state can be deactivated for a predetermined period of time of, for example, 30 minutes upon receiving a user input.

Further, different physiological parameter sensors can be used in addition or in alternative to the heart rate sensor of the present embodiment, such as transcutaneous O₂ or transcutaneous CO₂ sensors.

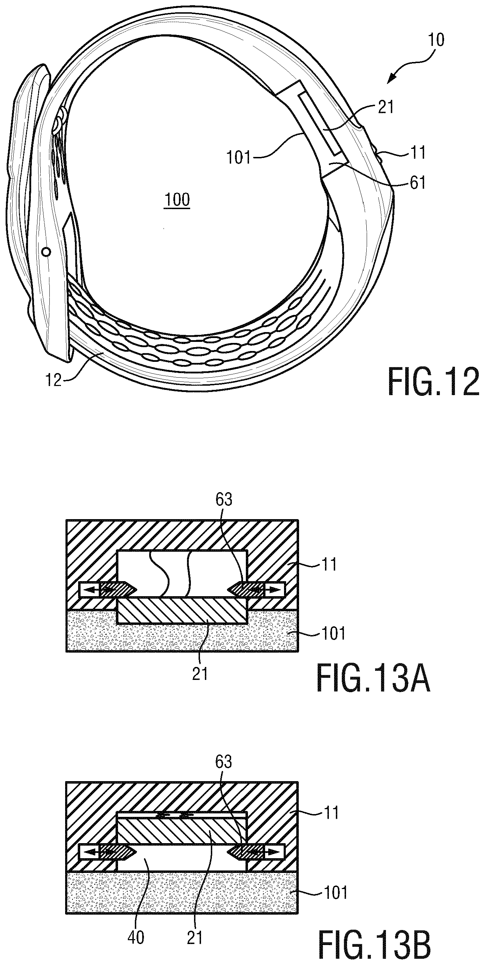

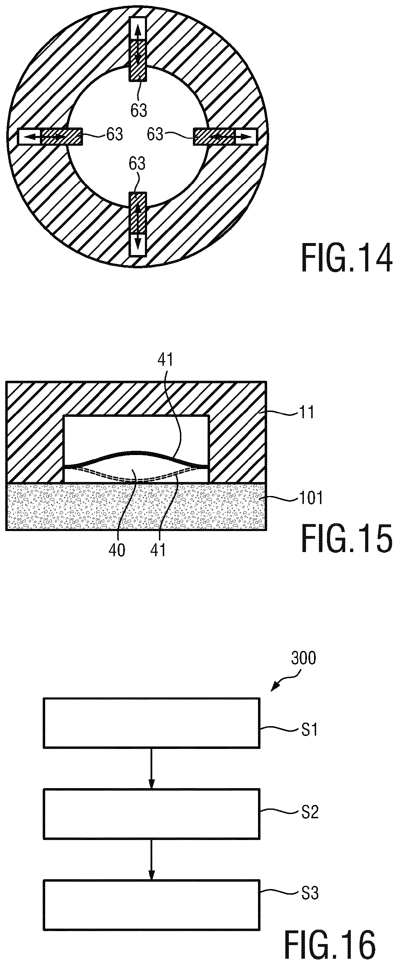

Fig. 12 shows a further embodiment of wearable device 10 comprising a housing 11 and a fixation element 12 for fixing the wearable device to the user 100. The device shown in Fig. 12 can comprise similar and/or identical features as the previous embodiments. Only differences will be highlighted. In the embodiment shown in Fig. 12, a recess 61 is provided within the housing 11. Instead of providing a spacing element in form of a membrane 41 as in the previous embodiments, the wearable device 10 is adapted such that the physiological parameter sensor 21 can be lifted away from the skin 101 of the subject 100 to provide a spacing 40 between said physiological parameter sensor 21 and the skin 101 of the subject. During measurement, the physiological parameter sensor 21 can be lowered towards and in contact with the skin 101 of the subject 100, as illustrated in Fig. 13A. Hence, the wearable device is configured to adopt a contact state, wherein the physiological parameter sensor on the lower side of the wearable device 10 is configured to contact the skin 101 of the subject 100. Fig. 13B illustrates the ventilation state wherein a spacing 40 is provided between the physiological parameter sensor 21 on the lower side of the wearable device 10 and the skin 101 of the subject 100. Optionally, fixing elements 63 can be provided to fix the physiological parameter sensor 21 in the respective positions for the ventilation state as illustrated in Fig. 13B and the contact state as illustrated in Fig. 13A. Fig. 14 shows a top sectional view illustrating a plurality of said fixing elements 63.

Fig. 15 shows yet another embodiment of a spacing element 41 that serves to provide a spacing 40 between the wearable device 10 and the skin 101 of the subject. The spacing element 41 as shown in Fig. 15 is adapted to provide a spacing 40 between the wearable device 10 and the skin 101 of the subject, wherein the spacing element is displaceable between a first, ventilation state, as indicated by the solid lines of the spacing element 41 in Fig. 15 and a second contact state, as illustrated by the dashed-lines of the spacing element 41 in Fig. 15. An advantage of this embodiment is that it is bi-stable. The spacing element is adapted to be switched, with a (small) force, from one state to the other and to stay in a stable position there.

In yet another embodiment, one or more spacing elements 41 can be provided in proximity of a physiological parameter sensor in order to lift the physiological parameter sensor from the skin of the user to provide a ventilation state; and to lower the physiological parameter sensor to the skin of the user to provide a contact state.

Fig. 16 shows a flow chart of an exemplary embodiment of a method according to an aspect of the present disclosure. Fig. 16 describes a method 300 for operating a wearable device, the wearable device comprising a fixation element for fixing the wearable device to a user, and a lower side for contacting a skin of the user when worn.

In a first step S1, the wearable device is fixed or applied to the user. In a second step S2, a sweat level measure indicative of an amount of sweat accumulated between the lower side of the wearable device and a skin of the user is determined by a processing unit. In a next step S3, a moment in time for ventilating said lower side of the wearable device is determined based on said sweat level measure by the processing unit.

While the invention has been described with reference to the embodiment of a wrist-worn wearable device, it shall be understood that a wearable device can also take other forms or may applied to other portions of the body of the user, such as a finger, an earlobe, an upper arm or the chest.

In conclusion, the wearable device, system and method presented herein enable an improved wearing comfort, in particular by enabling reduced skin irritation caused by sweat. Furthermore, the measurement accuracy can advantageously be improved since the removal of sweat can enable more accurate optical measurements due to reduced reflections and/or more accurate bio-impedance measurements since an impedance-influence due to a sweat layer on top of the skin can be reduced.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Wearable device (10) adapted to be worn by a user (100), the wearable device comprising
- a fixation element (12) for fixing the wearable device to the user;
- a lower side (15) for contacting a skin (101) of the user when worn; and
- a processing unit (20) adapted to determine a sweat level measure (32)
indicative of an amount of sweat accumulated between the lower side (15) of the wearable device and the skin (101) of the user and indicative of an exposure to sweat over time; and to determine a moment in time for ventilating said lower side of the wearable device based on a said sweat level measure (32).

2. Wearable device according to claim 1, further comprising at least one physiological parameter sensor (21) other than a skin conductance sensor for acquiring a physiological signal (31) of the user (100), and where the processing unit (20) is adapted to determine the sweat level measure (32) based on said physiological signal.

3. Wearable device according to any preceding claim, further comprising an activity sensor, and wherein the processing unit (20) is adapted to determine the sweat level measure (32) based on an output of said activity sensor.

4. Wearable device according to any preceding claim, further comprising a timer (22), and wherein the processing unit (20) is adapted to determine the sweat level measure (32) based on an output of said timer.

5. Wearable device according to any preceding claim, wherein the processing unit (20) is adapted to determine a moment in time for ventilating said lower side of the wearable device based on said sweat level measure and further taking into account when a continuous measurement of a physiological parameter is not needed, in particular wherein the processing unit is adapted to determine a moment in time for ventilating said lower side of the wearable device under the condition that a physiological parameter is stable.

6. Wearable device according to any preceding claim, further comprising an output unit (13), wherein the wearable device (10) is further adapted to provide a notification about said moment in time for ventilating said lower side (15) of the wearable device (10) via said output unit (13).

7. Wearable device according to any preceding claim, wherein the wearable device (10) is configured to adopt
- a ventilation state, wherein a spacing (40) is provided between at least a portion of said lower side (15) of the wearable device (10) and the skin (101) of the subject (100); and
- a contact state, wherein said portion of the lower side (15) of the wearable device (10) is configured to contact the skin (101) of the subject (100);
wherein the wearable device is further configured to oscillate between said ventilation state and said contact state when the processing unit determines the moment in time for ventilating said lower side of the wearable device.

8. Wearable device according to any preceding claim, wherein the wearable device (10) is configured to adopt a ventilation state, wherein a spacing (40) is provided between at least a portion of said lower side (15) of the wearable device (10) and the skin (101) of the subject (100); and a contact state, wherein said portion of the lower side (15) of the wearable device (10) is configured to contact the skin (101) of the subject (100);
wherein the wearable device further comprises a physiological parameter sensor for acquiring a physiological signal of the user, and
wherein the wearable device is adapted to switch to a different sensor or to a different measurement mode of the physiological parameter sensor in said ventilation state than in said contact state.

9. Wearable device according to any preceding claim, wherein the wearable device (10) is configured to adopt a ventilation state, wherein a spacing (40) is provided between at least a portion of said lower side (15) of the wearable device (10) and the skin (101) of the subject (100); and a contact state, wherein said portion of the lower side (15) of the wearable device (10) is configured to contact the skin (101) of the subject (100); wherein the wearable device (10) further comprises a spacing element (41) adapted to provide said spacing (40) between the wearable device (10) and the skin (101) of the subject (100), wherein the spacing element (41) is displaceable between said first, ventilation state and said second, contact state, wherein the spacing element is arranged between the lower side (15) and the skin (101) of the user (100) when worn and wherein, in the first, ventilation state, the spacing element is at least partially distanced from the lower side (15) of the wearable device (10), and in the second, contact state, the spacing element lies against the lower side (15) of the wearable device (10).

10. Wearable device according to claim 9, wherein the spacing element (41) is permeable to moisture.

11. Wearable device according to claim 9 or 10, further comprising a physiological parameter sensor (21) for acquiring a physiological signal (32) of the user (100); wherein the physiological parameter sensor is an optical physiological parameter sensor; and the wherein the spacing element (41) is transparent at a wavelength used by said optical physiological parameter sensor.

12. Wearable device according to any of claims 9 to 11, wherein at least one physiological parameter sensor is integrated in the spacing element.

13. System (1) comprising
- a wearable device (10') adapted to be worn by a user (100), and
- a processing unit (20'),
wherein the wearable device comprises:
- a fixation element (12) for fixing the wearable device (10') to the user (100); and
- a lower side (15) for contacting a skin (101) of the user (100) when worn;
wherein the processing unit (20') is adapted to determine a sweat level measure (32), indicative of an amount of sweat accumulated between the lower side (15) of the wearable device (10) and the skin (101) of the user (100) and indicative of an exposure to sweat over time; and to determine a moment in time for ventilating said lower side (15) of the wearable device (10) based on a said sweat level measure (32).

14. Method (300) for operating a wearable device (10), the wearable device comprising a fixation element (12) for fixing the wearable device to a user (100), and a lower side (15) for contacting a skin (101) of the user when worn; the method comprising the steps of:
- fixing the wearable device to the user (S1);
- determining, by a processing unit, a sweat level measure indicative of an amount of sweat accumulated between the lower side of the wearable device and the skin of the user and indicative of an exposure to sweat over time (S2); and
- determining, by the processing unit, a moment in time for ventilating said lower side of the wearable device based on a said sweat level measure (S3).

15. Computer program comprising program code means for causing a computer to carry out the steps of determining a sweat level measure indicative of an amount of sweat accumulated between the lower side of a wearable device fixed to a user and the skin of the user (S2) and indicative of an exposure to sweat over time; and determining a moment in time for ventilating said lower side of the wearable device based on a said sweat level measure (S3); when said computer program is carried out on a computer.

## Patentansprüche

1. Tragbare Vorrichtung (10), angepasst, um von einem Benutzer (100) getragen zu werden, wobei die tragbare Vorrichtung Folgendes umfasst:
- ein Befestigungselement (12) zum Befestigen der tragbaren Vorrichtung an dem Benutzer;
eine Unterseite (15) zum In-Kontakt-Treten mit einer Haut (101) des Benutzers, wenn getragen; und
eine Verarbeitungseinheit (20), die angepasst ist, um ein Schweißkonzentrationsmaß (32) zu bestimmen, das für eine zwischen der Unterseite (15) der tragbaren Vorrichtung und der Haut (101) des Benutzers angesammelte Menge an Schweiß indikativ ist und für eine Exposition gegenüber Schweiß im Verlauf der Zeit indikativ ist; und um einen Zeitpunkt zum Belüften der Unterseite der tragbaren Vorrichtung basierend auf dem Schweißkonzentrationsmaß (32) zu bestimmen.

2. Tragbare Vorrichtung nach Anspruch 1, weiter umfassend mindesten einen anderen physiologischen Parametersensor (21) als einen Hautleifähigkeitssensor zum Erfassen eines physiologischen Signals (31) des Benutzers (100), und wobei die Verarbeitungseinheit (20) angepasst ist, um das Schweißkonzentrationsmaß (32) basierend auf dem physiologischen Signal zu bestimmen.

3. Tragbare Vorrichtung nach einem vorhergehenden Anspruch, weiter umfassend einen Aktivitätssensor, und wobei die Verarbeitungseinheit (20) angepasst ist, um das Schweißkonzentrationsmaß (32) basierend auf einer Ausgabe des Aktivitätssensors zu bestimmen.

4. Tragbare Vorrichtung nach einem vorhergehenden Anspruch, weiter umfassend einen Timer (22), und wobei die Verarbeitungseinheit (20) angepasst ist, um das Schweißkonzentrationsmaß (32) basierend auf einer Ausgabe des Timers zu bestimmen.

5. Tragbare Vorrichtung nach einem vorhergehenden Anspruch, wobei die Verarbeitungseinheit (20) angepasst ist, um basierend auf dem Schweißkonzentrationsmaß einen Zeitpunkt zu bestimmen, um die Unterseite der tragbaren Vorrichtung zu belüften und um weiter zu berücksichtigen, wenn eine kontinuierliche Messung eines physiologischen Parameters nicht notwendig ist, wobei die Verarbeitungseinheit insbesondere angepasst ist, um einen Zeitpunkt zum Belüften der Unterseite der tragbaren Vorrichtung unter der Bedingung, dass ein physiologischer Parameter stabil ist, zu bestimmen.

6. Tragbare Vorrichtung nach einem vorhergehenden Anspruch, weiter umfassend eine Ausgabeeinheit (13), wobei die tragbare Vorrichtung (10) weiter angepasst ist, um eine Benachrichtigung über den Zeitpunkt zum Belüften der Unterseite (15) der tragbaren Vorrichtung (10) mittels der Ausgabeeinheit (13) bereitzustellen.

7. Tragbare Vorrichtung nach einem vorhergehenden Anspruch, wobei die tragbare Vorrichtung (10) konfiguriert ist, um Folgendes anzunehmen:
- einen Belüftungszustand, wobei zwischen mindestens einem Abschnitt der Unterseite (15) der tragbaren Vorrichtung (10) und der Haut (101) des Subjekts (100) ein Abstand (40) bereitgestellt ist; und
- einen Kontaktzustand, wobei der Abschnitt der Unterseite (15) der tragbaren Vorrichtung (10) konfiguriert ist, um mit der Haut (101) des Subjekts (100) in Kontakt zu treten;
wobei die tragbare Vorrichtung weiter konfiguriert ist, um zwischen dem Belüftungszustand und dem Kontaktzustand zu pendeln, wenn die Verarbeitungseinheit den Zeitpunkt zum Belüften der Unterseite der tragbaren Vorrichtung bestimmt.

8. Tragbare Vorrichtung nach einem vorhergehenden Anspruch, wobei die tragbare Vorrichtung (10) konfiguriert ist, um einen Belüftungszustand anzunehmen, wobei zwischen mindestens einem Abschnitt der Unterseite (15) der tragbaren Vorrichtung (10) und der Haut (101) des Subjekts (100) ein Abstand (40) bereitgestellt ist; und einen Kontaktzustand, wobei der Abschnitt der Unterseite (15) der tragbaren Vorrichtung (10) konfiguriert ist, um mit der Haut (101) des Subjekts (100) in Kontakt zu treten;
wobei die tragbare Vorrichtung weiter einen physiologischen Parametersensor zum Erfassen eines physiologischen Signals des Benutzers umfasst, und
wobei die tragbare Vorrichtung angepasst ist, um im Belüftungszustand auf einen anderen Sensor oder einen anderen Messmodus des physiologischen Parametersensors umzuschalten als im Kontaktzustand.

9. Tragbare Vorrichtung nach einem vorhergehenden Anspruch, wobei die tragbare Vorrichtung (10) konfiguriert ist, um einen Belüftungszustand anzunehmen, wobei zwischen mindestens einem Abschnitt der Unterseite (15) der tragbaren Vorrichtung (10) und der Haut (101) des Subjekts (100) ein Abstand (40) bereitgestellt ist; und einen Kontaktzustand, wobei der Abschnitt der Unterseite (15) der tragbaren Vorrichtung (10) konfiguriert ist, um mit der Haut (101) des Subjekts (100) in Kontakt zu treten; wobei die tragbare Vorrichtung (10) weiter ein Abstandselement (41) umfasst, das angepasst ist, um den Abstand (40) zwischen der tragbaren Vorrichtung (10) und der Haut (101) des Subjekts (100) bereitzustellen,
wobei das Abstandselement (41) zwischen dem ersten Zustand, dem Belüftungszustand, und dem zweiten Zustand, dem Kontaktzustand, verschiebbar ist, wobei das Abstandselement zwischen der Unterseite (15) und der Haut (101) des Benutzers (100) angeordnet ist, wenn getragen, und wobei im ersten Zustand, dem Belüftungszustand, das Abstandselement mindestens teilweise von der Unterseite (15) der tragbaren Vorrichtung (10) distanziert ist und im zweiten Zustand, dem Kontaktzustand, das Abstandselement an der Unterseite (15) der tragbaren Vorrichtung (10) anliegt.

10. Tragbare Vorrichtung nach Anspruch 9, wobei das Abstandselement (41) für Feuchtigkeit durchlässig ist.

11. Tragbare Vorrichtung nach Anspruch 9 oder 10, weiter umfassend einen physiologischen Parametersensor (21) zum Erfassen eines physiologischen Signals (32) des Benutzers (100); wobei der physiologische Parametersensor ein optischer physiologischer Parametersensor ist; und wobei das Abstandselement (41) bei einer Wellenlänge, die von dem optischen physiologischen Parametersensor verwendet wird, lichtdurchlässig ist.

12. Tragbare Vorrichtung nach einem der Ansprüche 9 bis 11, wobei mindestens ein physiologischer Parametersensor in dem Abstandselement integriert ist.

13. System (1), umfassend:
- eine tragbare Vorrichtung (10'), angepasst, um von einem Benutzer (100) getragen zu werden, und
- eine Verarbeitungseinheit (20'),
wobei die tragbare Vorrichtung Folgendes umfasst:
- ein Befestigungselement (12) zum Befestigen der tragbaren Vorrichtung (10') an dem Benutzer (100); und
- eine Unterseite (15) zum In-Kontakt-Treten mit einer Haut (101) des Benutzers (100), wenn getragen;
wobei die Verarbeitungseinheit (20') angepasst ist, um ein Schweißkonzentrationsmaß (32) zu bestimmen, das für eine zwischen der Unterseite (15) der tragbaren Vorrichtung (10) und der Haut (101) des Benutzers (100) angesammelte Menge an Schweiß indikativ ist und für eine Exposition gegenüber Schweiß im Verlauf der Zeit indikativ ist; und um einen Zeitpunkt zum Belüften der Unterseite (15) der tragbaren Vorrichtung (10) basierend auf dem Schweißkonzentrationsmaß (32) zu bestimmen.

14. Verfahren (300) zum Bedienen einer tragbaren Vorrichtung (10), wobei die tragbare Vorrichtung ein Befestigungselement (12) zum Befestigen der tragbaren Vorrichtung an einem Benutzer (100) und eine Unterseite (15) zum In-Kontakt-Treten mit einer Haut (101) des Benutzers, wenn getragen, umfasst; wobei das Verfahren die folgenden Schritte umfasst:
- Befestigen der tragbaren Vorrichtung an dem Benutzer (S1);
- Bestimmen, mittels einer Verarbeitungseinheit, eines Schweißkonzentrationsmaßes, das für eine zwischen der Unterseite der tragbaren Vorrichtung und der Haut des Benutzers angesammelte Menge an Schweiß indikativ ist und für eine Exposition gegenüber Schweiß im Verlauf der Zeit indikativ ist (S2); und
- Bestimmen, mittels der Verarbeitungseinheit, eines Zeitpunkts zum Belüften der Unterseite der tragbaren Vorrichtung basierend auf dem Schweißkonzentrationsmaß (S3).

15. Computerprogramm, umfassend Programmcodemittel, um zu bewirken, dass ein Computer die Schritte des Bestimmens eines Schweißkonzentrationsmaßes, das für eine zwischen der Unterseite der an dem Benutzer befestigten tragbaren Vorrichtung und der Haut des Benutzers angesammelte Menge an Schweiß indikativ ist (S2) und für eine Exposition gegenüber Schweiß im Verlauf der Zeit indikativ ist, und des Bestimmens eines Zeitpunkts zum Belüften der Unterseite der tragbaren Vorrichtung basierend auf dem Schweißkonzentrationsmaß, ausführt (S3), wenn das Computerprogramm auf einem Computer ausgeführt wird.

## Revendications

1. Dispositif portable (10) adapté à être porté par un utilisateur (100), le dispositif portable comprenant
- un élément de fixation (12) pour fixer le dispositif portable à l'utilisateur ;
- un côté inférieur (15) qui entre en contact avec la peau (101) de l'utilisateur lorsqu'il est porté ; et
- une unité de traitement (20) adaptée à déterminer une mesure du niveau de transpiration (32) indiquant une quantité de transpiration accumulée entre le côté inférieur (15) du dispositif portable et la peau (101) de l'utilisateur et indiquant une exposition à la transpiration dans le temps ; et à déterminer un moment dans le temps pour la ventilation dudit côté inférieur du dispositif portable sur la base de ladite mesure du niveau de transpiration (32).

2. Dispositif portable selon la revendication 1, comprenant en outre au moins un capteur de paramètre physiologique (21) autre qu'un capteur de conductance cutanée pour acquérir un signal physiologique (31) de l'utilisateur (100), et dans lequel l'unité de traitement (20) est adaptée à déterminer la mesure du niveau de transpiration (32) sur la base dudit signal physiologique.

3. Dispositif portable selon l'une quelconque des revendications précédentes, comprenant en outre un capteur d'activité, et dans lequel l'unité de traitement (20) est adaptée à déterminer la mesure du niveau de transpiration (32) sur la base d'une sortie dudit capteur d'activité.

4. Dispositif portable selon l'une quelconque des revendications précédentes, comprenant en outre un minuteur (22), et dans lequel l'unité de traitement (20) est adaptée à déterminer la mesure du niveau de transpiration (32) sur la base d'une sortie dudit minuteur.

5. Dispositif portable selon l'une quelconque des revendications précédentes, dans lequel l'unité de traitement (20) est adaptée à déterminer un moment dans le temps pour ventiler ledit côté inférieur du dispositif portable sur la base de ladite mesure du niveau de transpiration et en outre prenant en considération lorsqu'une mesure continue d'un paramètre physiologique n'est pas nécessaire, en particulier dans lequel l'unité de traitement est adaptée à déterminer un moment dans le temps pour la ventilation dudit côté inférieur du dispositif portable à condition qu'un paramètre physiologique soit stable.

6. Dispositif portable selon l'une quelconque des revendications précédentes, comprenant en outre une unité de sortie (13), dans lequel le dispositif portable (10) est en outre adapté à fournir une notification relative au dit moment dans le temps pour ventiler ledit côté inférieur (15) du dispositif portable (10) via ladite unité de sortie (13).

7. Dispositif portable selon l'une quelconque des revendications précédentes, dans lequel le dispositif portable (10) est configuré pour adopter
- un état de ventilation, dans lequel un espacement (40) est fourni entre au moins une partie dudit côté inférieur (15) du dispositif portable (10) et la peau (101) du sujet (100) ; et
- un état de contact, dans lequel ladite partie dudit côté inférieur (15) du dispositif portable (10) est configurée pour venir en contact avec la peau (101) du sujet (100) ;
dans lequel le dispositif portable est en outre configuré pour osciller entre ledit état de ventilation et ledit état de contact lorsque l'unité de traitement détermine le moment dans le temps pour la ventilation dudit côté inférieur du dispositif portable.

8. Dispositif portable selon l'une quelconque des revendications précédentes, dans lequel le dispositif portable (10) est configuré pour adopter un état de ventilation, dans lequel un espacement (40) est fourni entre au moins une partie dudit côté inférieur (15) du dispositif portable (10) et la peau (101) du sujet (100) ; et un état de contact, dans lequel ladite partie dudit côté inférieur (15) du dispositif portable (10) est configurée pour venir en contact avec la peau (101) du sujet (100) ;
dans lequel le dispositif portable comprend en outre un capteur de paramètres physiologiques pour l'acquisition d'un signal physiologique de l'utilisateur, et
dans lequel le dispositif portable est adapté à se commuter sur un capteur différent ou un mode de mesure différent du capteur de paramètres physiologiques dans ledit état de ventilation par rapport au dit état de contact.

9. Dispositif portable selon l'une quelconque des revendications précédentes, dans lequel le dispositif portable (10) est configuré pour adopter un état de ventilation, dans lequel un espacement (40) est fourni entre au moins une partie dudit côté inférieur (15) du dispositif portable (10) et la peau (101) du sujet (100) ; et un état de contact, dans lequel ladite partie dudit côté inférieur (15) du dispositif portable (10) est configurée pour venir en contact avec la peau (101) du sujet (100) ; dans lequel le dispositif portable (10) comprend en outre un élément d'espacement (41) adapté à permettre ledit espacement (40) entre le dispositif portable (10) et la peau (101) du sujet (100), dans lequel l'élément d'espacement (41) est déplaçable entre ledit premier état de ventilation et ledit second état de contact, dans lequel l'élément d'espacement est agencé entre le côté inférieur (15) et la peau (101) de l'utilisateur (100) lorsqu'il est porté et dans lequel, dans le premier état de ventilation, l'élément d'espacement est au moins partiellement écarté du côté inférieur (15) du dispositif portable (10), et dans le second état de contact, l'élément d'espacement repose contre le côté inférieur (15) du dispositif portable (10).

10. Dispositif portable selon la revendication 9, dans lequel l'élément d'espacement (41) est perméable à l'humidité.

11. Dispositif portable selon la revendication 9 ou 10, comprenant en outre un capteur de paramètres physiologiques (21) pour l'acquisition d'un signal physiologique (32) de l'utilisateur (100) ; dans lequel le capteur de paramètres physiologiques est un capteur de paramètres physiologiques optiques ; et dans lequel l'élément d'espacement (41) est transparent à une longueur d'onde utilisée par ledit capteur de paramètres physiologiques optiques.

12. Dispositif portable selon l'une quelconque des revendications 9 à 11, dans lequel au moins un capteur de paramètres physiologiques est intégré dans l'élément d'espacement.

13. Système (1) comprenant
- un dispositif portable (10') adapté à être porté par un utilisateur (100), et
- une unité de traitement (20'),
dans lequel le dispositif portable comprend :
- un élément de fixation (12) pour fixer le dispositif portable (10') à l'utilisateur (100) ; et
- un côté inférieur (15) qui entre en contact avec la peau (101) de l'utilisateur (100) lorsqu'il est porté ;
dans lequel l'unité de traitement (20') est adaptée à déterminer une mesure du niveau de transpiration (32), indiquant une quantité de transpiration accumulée entre le côté inférieur (15) du dispositif portable (10) et la peau (101) de l'utilisateur (100) et indiquant une exposition à la transpiration dans le temps ; et à déterminer un moment dans le temps pour la ventilation dudit côté inférieur (15) du dispositif portable (10) sur la base de ladite mesure du niveau de transpiration (32).

14. Procédé (300) de fonctionnement d'un dispositif portable (10), le dispositif portable comprenant un élément de fixation (12) pour fixer le dispositif portable à un utilisateur (100), et un côté inférieur (15) qui entre en contact avec la peau (101) de l'utilisateur lorsqu'il est porté ; le procédé comprenant les étapes de :
- fixation du dispositif portable à l'utilisateur (S1) ;
- détermination, par une unité de traitement, d'une mesure du niveau de transpiration indiquant une quantité de transpiration accumulée entre le côté inférieur du dispositif portable et la peau de l'utilisateur et indiquant une exposition à la transpiration dans le temps (S2) ; et
- détermination, par l'unité de traitement, d'un moment dans le temps pour la ventilation dudit côté inférieur du dispositif portable sur la base de ladite mesure du niveau de transpiration (S3).

15. Programme informatique comprenant un moyen de code de programme pour amener un ordinateur à réaliser les étapes de détermination d'une mesure du niveau de transpiration indiquant une quantité de transpiration accumulée entre le côté inférieur d'un dispositif portable fixé sur un utilisateur et la peau de l'utilisateur (S2) et indiquant une exposition à la transpiration dans le temps ; et déterminer un moment dans le temps pour la ventilation dudit côté inférieur du dispositif portable sur la base de ladite mesure du niveau de transpiration (S3) ; lorsque ledit programme informatique est réalisé sur un ordinateur.
